# EUROPEAN PATENT APPLICATION

(11) **EP 2 138 148 A1**
(43) Date of publication of application: **30.12.2009**
(21) Application number: 07827984.1
(22) Date of filing: 05.11.2007
(51) Int. Cl.: A61J 1/05, A61J 1/06, A61M 5/30

(54) **MECHANISM CAPABLE OF PROVIDING NEAT CLEAVED OPENING SURFACE AND AMPULE WITH MOVABLE GASKET**

(30) Priority: 07.11.2006 JP 2006302170
(71) Applicant: Oyama, Yoshio, Kawasaki-shi Kanagawa 216-0035 (JP)
(72) Inventor: Oyama, Yoshio, Kawasaki-shi Kanagawa 216-0035 (JP)
(74) Representative: Schweiger, Martin
(86) International application number: PCT/JP2007/001206
(87) International publication number: WO 2008/056442

(57) **Abstract**

[Problem] An object of the present invention is to provide an ampoule which is hard to crack on a barrel while increasing cleavability required for a hermetical container end.

[Means for Solving Problem] The above object is solved by an ampoule (20) provided with a front edge portion (1), a main body (2), a connection (3), and a joint (4), wherein a hermetical container end (10) which includes the front edge portion (1), the joint (4) and the connection (3) is formed separately from the main body (2), and the joint (4) breaks when a force is applied from a lateral direction with respect to the central axis (5), so that a head portion of the connection (3) which is circular truncated cone shaped is exposed and the space portion (13) of the connection (3) becomes an open end.

## Description

### [Technical Field]

The present invention relates to an ampoule whose hermetical container end and main body are respectively formed of different materials. More specifically, the present invention relates to an ampoule usable as a syringe for a needleless injection, and the like.

### [Background Art]

Generally, many of drugs are orally administered. However, as for insulin for treating diabetes, interferon for treating hepatitis C or cancer, and the like, a patient himself or herself administers the drug using an injection syringe. Treating methods using such a form of administration require continuous administration of injectable solution. Therefore, the patient has daily suffered from fear of the syringe needle, ache upon administration of the injectable solution, or the like, resulting in great mental burden.

Therefore, needleless syringes with no needles have been developed and used in recent years as substitutes for syringes with needles. A needleless syringe shoots the drug solution at an extremely high speed towards the skin. As a result, a small hole is made at the skin, so that the drug solution penetrates through the skin. Accordingly, the drug solution can be administered (see for example, Japanese Patent Application Laid-Open Publication No. 2003-093508 (Patent Citation 1)).

However, every time the above-mentioned needleless syringe is used, it is required first to attach a special adapter to a vial. After a specified quantity of the drug solution is suctioned from the vial into the syringe barrel, this syringe barrel having suctioned the drug solution is reattached to an injector. Thereafter, the syringe is applied to a specified site for a shot, where the drug solution is pushed out at a high speed to be administered. Accordingly, the handling is complicated and it is not always easy to suction and administer an adequate amount. Moreover, since the same needleless syringe is used multiple times for suctioning the drug solution for administration, problems exist from a hygienic perspective such that viruses and the like may attach to the aperture plane and replicate. Therefore, preparation of hermetically sealed drug solution in an amount per single administration to be opened and administered per administration can be conceived.

In this case, preparation of a cleavable ampoule including an amount of drug solution for a single administration can be conceived. However, in case of a needleless syringe, a high pressure is applied upon use, so that a side face of the ampoule may be cracked or damaged.

Also, it is not easy to pour liquid inside the ampoule for the ampoule alone. Moreover, when an ampoule is used as a needleless syringe, it is required that a hole for emitting liquid is finely produced. However, there is a problem that such a molding is not always easy.
[Patent Citation 1] Japanese Patent Application Laid-Open Publication No. 2003-093508

### [Disclosure of Invention]

### [Problems to be solved by the Invention]

An object of the present invention is to provide an ampoule which is hard to crack on a barrel and hard to break while increasing cleavability required for a hermetical container end.

Another object of the present invention is to provide an ampoule capable of easily spraying liquid.

### [Means for solving the Problems]

The present invention is based on a knowledge that basically an ampoule which is hard to crack on a barrel and hard to break while increasing cleavability required for a hermetical container end can be obtained by molding the hermetical container end and the main body with different materials.

The first aspect of the present invention relates to an ampoule (20) comprising: a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3), and which is narrower than the front edge portion (1) and the connection (3), a hermetical container end (10) which includes the front edge portion (1), the joint (4) and the connection (3) being formed separately from the main body (2), the front edge portion(1), the joint (4), the connection (3) and the main body (2) being coaxially aligned to have a central axis (5), having sequential space portions (11, 14, 13, 12) extending from the main body (2) to a midway of the front edge portion (1), the joint (4) breaking when a force is applied from a lateral direction with respect to the central axis (5), so that a head portion of the connection (3) which is circular truncated cone shaped is exposed and the space portion (13) of the connection (3) becomes an open end.

In the expression "a front edge portion which is removed when the ampoule is used", "when the ampoule is used" means when drug solution or the like is administered using the ampoule. An ampoule is expected to have drug solution contained in its main body (2). Therefore, it has been a common practice to integrally form an ampoule. The present invention defies such a common practice by separately forming the hermetical container end and the main body to be assembled to form a single ampoule. This enables the hermetical container end to be given a property of fragility by using, for example, a highly crystalline resin or glass. On the other hand, the main body can be effectively prevented from cracking or breaking by using relatively elastic resin.

A preferred embodiment of the present invention is the ampoule (20) as described above, wherein the connection (3) of the hermetical container end (10) comprises: a hollow cylindrical insertion barrel (6) provided at a portion which is in contact with the main body (2); and a projection (7) provided on an inner circumferential surface of the insertion barrel (6), the main body (2) comprises: a first dent (8) into which the insertion barrel (6) is inserted; a second dent (9) into which the projection (7) is inserted, the insertion barrel (6) is inserted into the first dent, and the projection (7) is inserted into the second dent (9).

When the hermetical container end and the main body are separately formed, airtightness of the ampoule becomes an issue. However, since the insertion barrel (6) is inserted into the first dent (8), and the projection (7) is inserted into the second dent (9), the ampoule would have a high airtightness even if the hermetical container end and the main body are separately formed. Therefore, it is made possible to prevent a situation where a leak occurs at normal temperature and normal pressure.

A preferred embodiment of the present invention is the ampoule (20) as described in any one of the above, wherein; the connection (3) of the hermetical container end (10) comprises: a first hollow cylindrical insertion barrel (21) provided at a portion which is in contact with the main body (2), a second hollow cylindrical insertion barrel (22) provided at a portion which is in contact with the main body (2), and having a diameter smaller than that of the first insertion barrel (21), and a dent provided at a base of the first insertion barrel (21) and the second insertion barrel (22); the main body (2) comprises: an insertion barrel (23) of the main body (2) which is inserted to the first insertion barrel (21) and the second insertion barrel (22); and an insertion barrel front edge portion (24) which is located at a front edge of the insertion barrel (23) of the main body (2) and fits into the dent. The insertion barrel (23) may be hollow cylindrical or may be shaped otherwise.

When the hermetical container end and the main body are separately formed, airtightness of the ampoule becomes an issue. However, since the insertion barrel (23) of the main body fits between the first insertion barrel (21) and the second insertion barrel (22) while the insertion barrel front edge portion (24) fits in a dent provided at a base of the first insertion barrel (21) and the second insertion barrel (22), the airtightness between the hermetical container end and the main body would become extremely high. Therefore, the ampoule would have a high airtightness even if the hermetical container end and the main body are separately formed, so that it is made possible to prevent a situation where a leak occurs at normal temperature and normal pressure.

A preferred embodiment of the present invention is the ampoule (20) according to any one of the above-mentioned ampoules, wherein the hermetical container end (10) has a higher cleavage than that of the main body (2), and the main body (2) has a higher plasticity than that of the hermetical container end (10). Due to such a property, the front edge portion of the hermetical container end (10) can be removed easily, and it is made possible to prevent a situation where the main body (2) cracks.

A preferred embodiment of the present invention is the ampoule (20) according to any one of the above-mentioned ampoules, wherein the front edge portion (1) of the hermetical container end (10) has a tip projection (1a) at a tip of the front edge portion (1) which fits into the space portion (14) located at the joint (4). Generally, an object of a syringe for a needleless injection is to subcutaneously administer a drug solution. On the other hand, there are cases where it is desired that aromatic substance or the like is accommodated within the ampoule so that the liquid can be widely diffused. In this embodiment, the front edge portion (1) has such a form that when the front edge portion (1) is removed from the hermetical container end (10), by inserting the tip projection of the front edge portion (1) into the space portion (13), an ampoule for diffusing liquid can be obtained.

### [Effects of the Invention]

According to the present invention, it is made possible to provide an ampoule which is hard to crack on a barrel while increasing cleavability required for a hermetical container end by separately forming the hermetical container end and the main body.

According to the present invention, front edge portion (1) of the hermetical container end (10) is made to have a tip projection (1a) which has a shape that fits in the space portion (14) located at the joint (4), so that an ampoule capable of easily spraying liquid can be provided.

### [Brief Description of the Drawings]

[Fig. 1] Fig. 1 is a schematic diagram showing a basic arrangement of an ampoule of the present invention. Fig. 1(a) shows a cross sectional view of an ampoule and Fig. 1(b) shows a cross sectional view of an ampoule accommodated in an ampoule guide.
[Fig. 2] Fig. 2 is an enlarged view of a joining portion between a hermetical container end and a main body.
[Fig. 3] Fig. 3 is an enlarged view of a joining portion between a hermetical container end and a main body.
[Fig. 4] Fig. 4 is a diagram showing an example of a cross sectional view of a hermetical container end of the present invention.
[Fig. 5] Fig. 5 is a diagram showing an example of a cross sectional view of a hermetical container end of the present invention. Fig. 5(a) shows a state before a front edge portion is removed and Fig. 5(b) shows a state after the front edge portion is removed.
[Fig. 6] Fig. 6 is a diagram showing an example of a cross sectional view of a hermetical container end of the present invention. Fig. 6(a) shows a state before a front edge portion is removed and Fig. 6(b) shows a state where the front edge portion is inserted into the joint after the front edge portion has been removed. Fig. 6 (c) shows the joint after the front edge portion is removed and Fig. 6(d) shows a state where the front edge portion is inserted into space portion located at the joint and rotated 90 degrees to be fixed.
[Fig. 7] Fig. 7 is a schematic diagram showing a basic arrangement of an ampoule guide and an ampoule.
[Fig. 8] Fig. 8 is an overview diagram of an assembled needleless syringe unit.
[Fig. 9] Fig. 9 is a diagram showing a basic arrangement of an administering device.
[Fig. 10] Fig. 10 shows an administering device after the front edge portion is removed.
[Fig. 11] Fig. 11 is a diagram showing an example of a needle-tipped syringe. Fig. 11(A) shows an example of a needle-tipped syringe using an ampoule having an ampoule guide. On the other hand, Fig. 11(B) shows a commercially available syringe to be used by inserting an ampoule inside the syringe.
[Fig. 12] Fig. 12 is a diagram showing an example of a sprayer (diffuser).

### [Explanation of Reference]

- 1: front edge portion
- 2: main body
- 3: connection
- 4: joint
- 5: central axis
- 11-14: space portion
- 20: ampoule

### [Best Mode for Carrying Out the Invention]

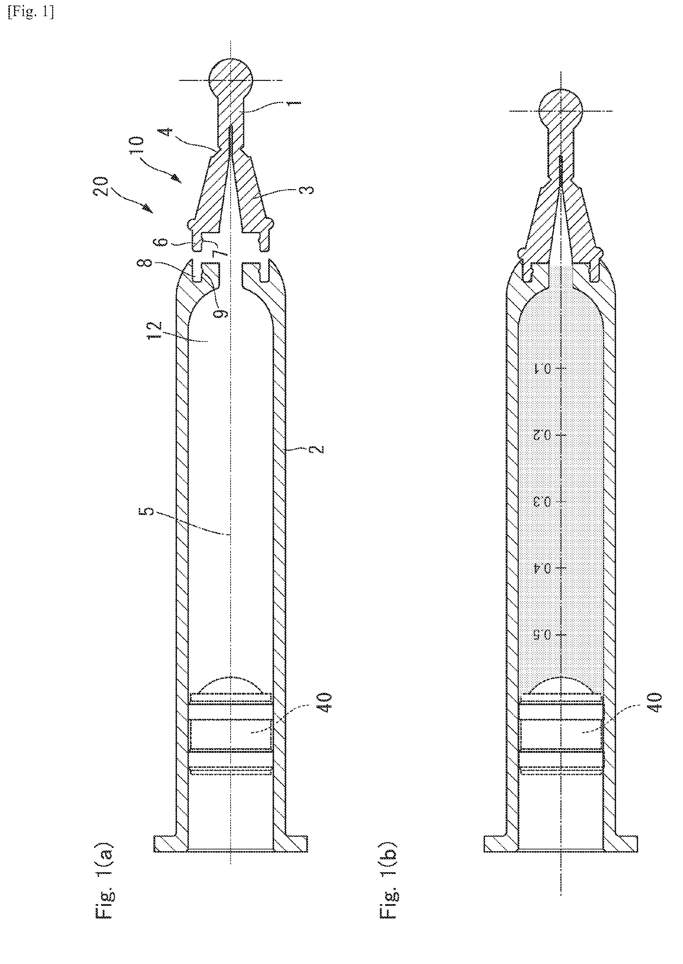

Hereinafter, an ampoule of the present invention will be described according to the drawings. Fig. 1 is a schematic diagram showing a basic arrangement of the ampoule of the present invention. Fig. 1(a) shows a sectional view of the ampoule, and Fig. 1(b) shows a sectional view of the ampoule accommodated in an ampoule guide. As shown in Fig. 1(a), the ampoule of the present invention is an ampoule (20) provided with a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3) and which is narrower than the front edge portion (1) and the connection (3) . A hermetical container end (10) which includes the front edge portion (1), the joint (4) and the connection (3) is formed separately from the main body (2). The front edge portion(1), the joint (4), the connection (3) and the main body (2) are coaxially aligned to have a central axis (5), having sequential space portions (11, 14, 13, 12) extending from the main body (2) to a midway of the front edge portion (1). The joint (4) breaks when a force is applied from a lateral direction with respect to the central axis (5), so that a head portion of the connection (3) which is circular truncated cone shaped is exposed and the space portion (13) of the connection (3) becomes an open end. It is to be noted that in Fig. 1(b), a reference numeral 30 denotes an ampoule guide and a reference numeral 31 denotes a hollow cylindrical guard.

An ampoule is expected to have drug solution contained in its main body (2). Therefore, it has been a common practice to integrally form an ampoule. The present invention defies such a common practice by separately forming the hermetical container end and the main body to be assembled to form a single ampoule. This enables the hermetical container end to be given a property of fragility by using, for example, a highly crystalline resin or glass. On the other hand, the main body can be effectively prevented from cracking or breaking by using relatively elastic resin. A preferred embodiment of the present invention is a cartridge type ampoule. Namely, after the ampoule is inserted into a needleless injection unit and used, the used ampoule is replaced with a new ampoule.

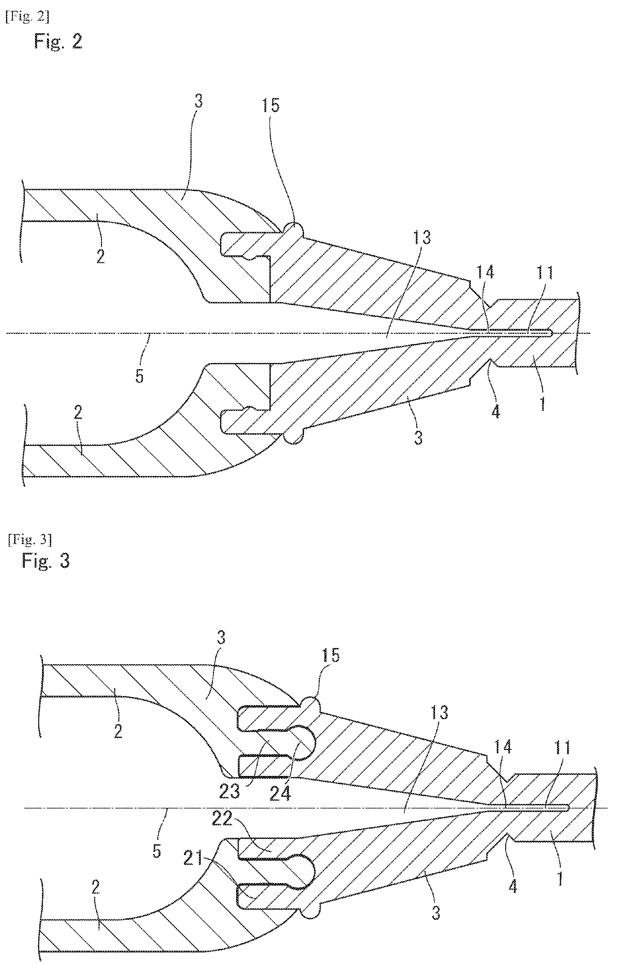

Fig. 2 is an enlarged view of a joining portion between the hermetical container end and the main body. As shown in Fig. 2, a preferred embodiment of the present invention is an ampoule according to any one of the above-mentioned ampoules wherein the connection (3) of the hermetical container end (10) is provided with a hollow cylindrical insertion barrel (6) provided at a portion which is in contact with the main body (2), and a projection (7) provided on an inner circumferential surface of the insertion barrel (6), and the main body (2) is provided with a first dent (8) into which the insertion barrel is inserted; a second dent (9) into which the projection (7) is inserted, the insertion barrel (6) is inserted into the first dent, and the projection (7) is inserted into the second dent (9). As a height of the insertion barrel (6), between 0.1 mm and 1 cm, both inclusive, can be mentioned in view of stability and ease to insert, while between 1 mm and 5 mm, both inclusive, is preferable. It is preferable that the insertion barrel (6) and the first dent (8) have approximately the same shape. Also, it is preferable that the shapes of the projection (7) and the second dent (9) are identical. It is preferable that the projection (7) is provided ring-like on an inner circumference of the insertion barrel (6).

When the hermetical container end and the main body are separately formed, airtightness of the ampoule may become an issue. However, since the insertion barrel (6) is inserted into the first dent (8) and the projection (7) is inserted into the second dent (9), the ampoule would have a high airtight ness even if the hermetical container end and the main body are separately formed. Therefore, it is made possible to prevent a situation where a leak occurs at normal temperature and normal pressures. It is to be noted that when the ampoule is used as a syringe for needleless injection, there is no problem in particular even if some leak occurs.

In the example shown in Fig. 2, an outer circumference of the connection (3) of the hermetical container end (10) is provided with a ring-like projection, which fits into a groove provided at the ampoule guide (30) to fix the ampoule. In a more preferable type of this embodiment, a projection provided on the outer circumference of the connection (3) is identical in shape to the dent of the guide. The injection syringe unit of such an embodiment is preferable since the stability thereof is increased. Moreover, the ring-like projection located at the end of the main body or within 5 mm (preferably 1 mm or less) from the end is preferable since it is made hard to apparently recognize that the hermetical container end and the main body are separately formed, so that sense of security can be provided to the users.

Fig. 3 is an enlarged view of a joining portion between the hermetical container end and the main body. As shown in Fig. 3, an ampoule of the following embodiment is preferable. Namely, a preferable embodiment of the present invention is an ampoule (20) according to any one of the above-mentioned ampoules wherein; the connection (3) of the hermetical container end (10) is provided with: a first hollow cylindrical insertion barrel (21) provided at a portion which is in contact with the main body (2), a second hollow cylindrical insertion barrel (22) provided at a portion which is in contact with the main body (2), and having a diameter less than that of the first insertion barrel (21), and a dent provided at a base of the first insertion barrel (21) and the second insertion barrel (22); and the main body (2) is provided with an insertion barrel (23) of the main body which is inserted to the first insertion barrel (21) and the second insertion barrel (22); and a insertion barrel front edge portion (24) which is located at a front edge of the insertion barrel (23) and fits into the dent. The form of the insertion barrel front edge portion (24) may be designed as appropriate taking into consideration the ease to insert and the fixing strength. As a cross sectional shape (cross sectional shape shown in Fig. 3), circular, semicircular, incomplete circular, elliptical, multangular shaped, triangular, rectangular, and the like can be mentioned, while an incomplete circular whose missing arc takes the width of the insertion barrel (23) of the main body as a cord as shown in Fig. 3 is preferable.

A preferred embodiment of the present invention is an ampoule (20) according to any one of the above-mentioned ampoules, wherein, the hermetical container end (10) has a higher cleavage than that of the main body (2), and the main body (2) has a higher plasticity than those of the hermetical container end (10). With such a property, the front edge portion of the hermetical container end (10) can be easily removed, and the situation where the main body (2) cracks can be prevented. It is to be noted that high-low degree of cleavage can be evaluated according to the method described in, for example, JISA1151. Also, by using two members to make test piece of an identical shape, and occasionally adding weight, the cleavage may be evaluated. Assuming the cleavage of the hermetical container end (10) A₁ and the cleavage of the main body (2) A₂, while it is effective even if A₁/A₂ is equal to or less than 1, it is difficult to achieve a preferable property, and if the properties are too much different, separate processing is required which leads to complication. Therefore, as a value of A₁/A₂, 1.1 to 1×10², both inclusive, can be mentioned, while 1.5 to 5x10 is acceptable, and 2 to 2x10 is also acceptable. If the value of A₁/A₂ is within this range, the hermetical container end (10) and the main body (2) can be manufactured using analogous compounds by changing a molecular weight or the like as will be described later. Therefore, the ampoule can be easily disposed of after use. Also, plasticity can be measured by a method described in JIS K 6546, for example.

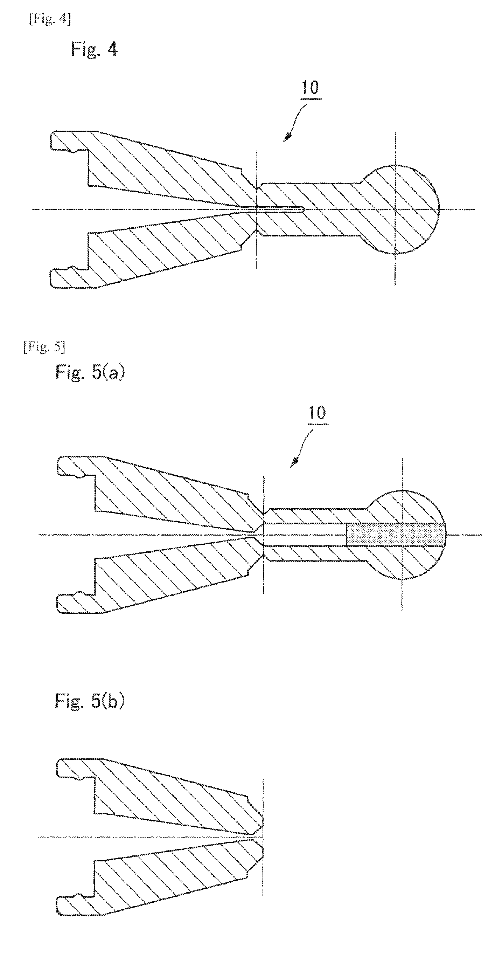

Fig. 4 is an example of a sectional view of a hermetical container end of the present invention. As shown in Fig. 4, the end of the front edge portion may have a sphere attached thereto. Due to such a shape, the joint is easy to break. It is not limited to such a sphere, but a ball with an elliptical cross section or polygonal shape can also be used. Fig. 5 is a diagram showing an example of a sectional view of the hermetical container end of the present invention. Fig. 5(a) shows a state before the front edge portion is removed and Fig. 5 (b) shows a state after the front edge portion is removed. As shown in Fig. 5(b), the hermetical container end may be shaped such that the inner diameter is increased towards the open end so that liquid within the ampoule will be diffused.

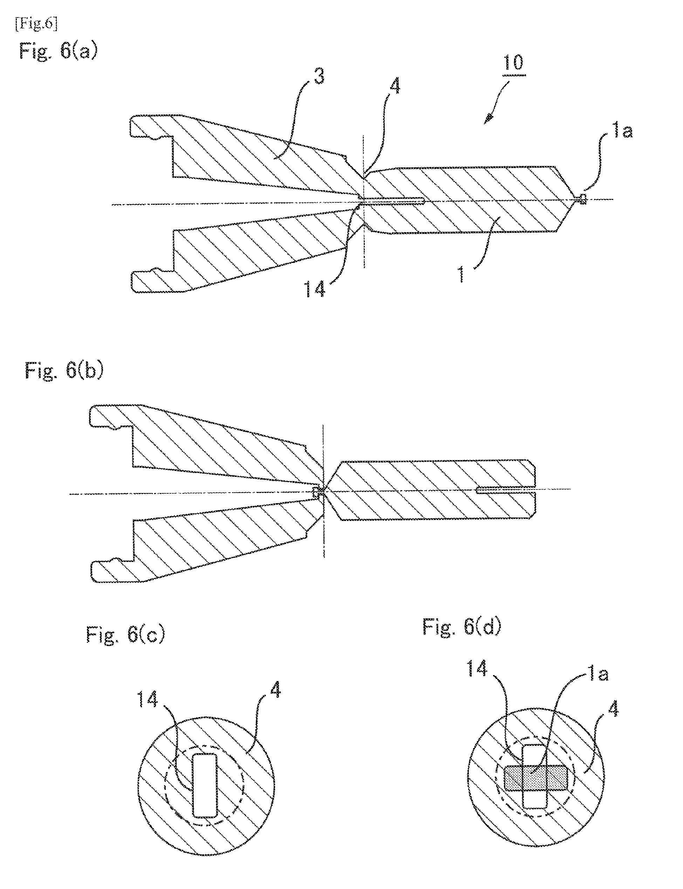

Fig. 6 is a diagram showing an example of a sectional view of the hermetical container end of the present invention. Fig. 6 (a) shows a state before the front edge portion is removed and Fig. 6(b) shows a state after the front edge portion is removed and then inserted into the joint. Fig. 6(c) shows the joint after the front edge portion is removed, and Fig. 6(d) shows how the front edge portion is inserted into the space portion located at the joint and rotated 90 degrees to be fixed. The ampoule shown in Fig. 6 has a tip projection (1a) which has a shape that fits in the space portion (14) located at the joint (4). As a shape of such a tip projection (1a), a tip which is long in a direction vertical to the axis (5) can be mentioned. After breaking the joint (4), the tip projection (1a) is inserted into the space portion (14) located at the joint (4). Since the space portion (14) also has a rectangular open end, the tip projection (1a) can be inserted into the open end. On the other hand, when the tip projection (1a) is rotated 90 degrees, the tip projection is shifted from the open end (14) by 90 degrees to be fixed as shown in Fig. 6(d). In this embodiment, the front edge portion (1) has such a shape that an ampoule for diffusing liquid can be obtained by removing the front edge portion (1) from the hermetical container end (10) and then inserting the tip projection of the front edge portion (1) into the space portion (13) of the connection.

In the ampoule shown in Fig. 1, the connection (3) is circular truncated cone shaped and the width of the joint (4) is narrowed, so that the joint can easily break by simply applying a lateral force to the front edge portion (1), and the broken surface becomes nearly planar. Thus a smooth and flat open end can be quickly and easily obtained. The space portion (14) of the joint (4) and the space portion (11) of the front edge portion (1) are preferably cylindrical, circular truncated cone shaped, or inverse circular truncated cone shaped which is coaxial to the central axis (5) and 1×10⁻¹ mm - 1 mm can be mentioned as its average diameter, while 1×10⁻¹ mm - 5×10⁻¹ is more preferable. Depth of entry or range of diffusion of the drug solution can be controlled by changing the shape or the average diameter.

As described later, the ampoule of the present invention is used by breaking the joint so as to obtain the open end. Therefore, sequential space portions (14, 13, 12) extending from the main body (2) to a midway of the joint (4) may be provided. However, considering the ease of manufacturing the hermetical container end, it is preferable to provide sequential space portions (11, 14, 13, 12) extending from the main body (2) to a midway of the front edge portion (1). In Fig. 1, the space portion (11) of the front edge portion and the space portion (14) of the joint are made into a sequential cylindrical portion, which may be shaped narrower towards the tip. As an angle of inclination for this narrowing (i.e. apex angle of the cone made by extending the portion which becomes narrower towards the tip), 1 degree - 45 degrees can be mentioned, while 5 degrees - 30 degrees is preferable, and 10 degrees - 20 degrees is more preferable, and 5 degrees - 10 degrees is also applicable. Such an angle enables adjustment of liquid current so as to increase the entry speed of the drug solution and deliver the drug to a deeper portion under the skin. On the other hand, the width may be made wider towards the tip, in which case, the angle may be reversed from the above-mentioned angle. In such a case, the entry speed of the liquid is slowed down as a result, but the liquid can be diffused to be administered to a relatively wide range. It is to be noted that the space portion (13) of the connection (3) becomes narrower towards the tip, and the angle (apex angle) may be made along the outer shape of the connection (3) . This will enable the strength to be uniform. Also, the space portion of the connection may be intentionally designed to have a different shape from the outer shape of the connection, for example, a portion shaped narrower towards the tip. As an angle of inclination for a portion thus shaped, 1 degree - 45 degrees can be mentioned, while 5 degrees - 30 degrees is preferable, and 10 degrees - 20 degrees is more preferable. Namely, in order to deliver the drug solution within the ampoule into the living body, it is desirable that a loss is small, and it is preferable to prevent as much as possible the situation where flow separation phenomenon occurs along the path. It is conceived from the hydrodynamic view point that the loss can be made small by making the apex angle (aperture) 15 degrees. On the other hand, with the ampoule of the present invention, if the drug solution is distributed to a wide area, pain points within the skin may be passed through, so that it may not be painless. From such a view point, the apex angle may be made 1 degree - 10 degrees, and preferably 5 degrees - 10 degrees. Also, the space portion (14) provided at the joint (4), or a portion connected to the space portion (14) within the space portion (14) and the space portion (13) of the connection (3) may have a cylindrical portion. In this case, assuming that the height (length) of the cylindrical portion is L and the diameter of the cross section is D, separated flow is generated in case L/D is equal to or greater than 5, while if L/D is small the loss of current is small but the pressure applied at the tip of the ampoule becomes high so that the ampoule may break. From such a point of view, L/D is preferably between 0.1 and 5, both inclusive, while it may be between 0.5 and 3, both inclusive, between 0.5 and 2, both inclusive, or between 1 and 2, both inclusive.

For the space portion (13) of the connection (3), one having a cylindrical portion which is connected to the space portion (11) of the front edge portion (1) and a circular truncated cone shaped portion whose head portion is connected to the cylindrical portion and bottom face is connected to the space portion (12) of the main body (2) can be mentioned. Accordingly, by intentionally providing the cylindrical portion, the flow of the drug solution can be controlled, so that the entering depth and the spread of the drug solution can be controlled. It is to be noted that the cylindrical portion in the connection (3) may be circular truncated cone shaped with a smaller apex angle of the circular truncated cone shape than that of the circular truncated cone shaped portion. For the height (length) of the cylindrical portion, one equal to or less than the height (length) of the circular truncated cone shaped portion can be mentioned. Namely, 10 % - 90 % of the height of the circular cylindrical truncated cone shaped portion (13b) can be mentioned as the height (length) of the cylindrical portion, while the percentage may be 20 % - 80% or 30 % - 70 %. The height of the cylindrical portion (13a) and the height of the circular truncated cone shaped portion (13b) are thus adjusted as appropriate, so that the flow of the drug solution can be controlled. Also, assuming that the height (length) of the cylindrical portion (13a) is L and the diameter of the cross section is D, separated flow is generated in case L/D is equal to or greater than 5, while if L/D is small the loss of current is small but the pressure applied at the tip of the ampoule becomes high so that the ampoule may break. From such a point of view, L/D is preferably between 0.1 and 5, both inclusive, while it may be between 0.5 and 3, both inclusive, between 0.5 and 2, both inclusive, or between 1 and 2, both inclusive.

For the space portion (13) of the connection (3), one having a cylindrical portion which is connected to the space portion (11) of the front edge portion (1) and a circular truncated cone shaped portion whose bottom face is connected to the space portion (12) of the main body (2) can be mentioned. For the cylindrical portion whose height (length) is equal to or greater than that of the circular truncated cone shaped portion, the height of the circular truncated cone shaped portion can be mentioned as 10 % - 90 % of the height of the cylindrical portion, while the percentage may be 20 % - 80% or 30 % - 70 %. The height of the cylindrical portion and the height of the circular truncated cone shaped portion are thus adjusted as appropriate, so that the flow of the drug solution can be controlled. It is to be noted that the cylindrical portion in the connection (3) may be circular truncated cone shaped with a smaller apex angle compared to that of the circular truncated cone shaped portion. Also, the cylindrical portion is preferably shaped as a circular truncated cone. Namely, in order to deliver the drug solution within the ampoule into the living body, it is desirable that a loss is small, and it is preferable to prevent as much as possible the situation where flow separation phenomenon occurs along the path. It is conceived from the hydrodynamic view point that the loss can be made small by making the apex angle (aperture) of the cylindrical portion (13a) approximately 15 degrees. On the other hand, with the ampoule of the present invention, if the drug solution is distributed to a wide area, pain points within the skin may be passed, so that it may not be painless. From such a view point, the apex angle may be made 1 degree - 10 degrees, and preferably 5 degrees - 10 degrees.

The space portion (13) of the connection (3) may include a plurality of independent cylindrical space portions. Specifically, one provided with two or three cylinders at symmetrical locations surrounding the central axis (5) is preferable. With such a shape, the drug solution can be administered diffusively, so that the damage to the skin can be suppressed to minimum. Also preferable is one provided with a single cylindrical or a circular truncated cone shaped (whether tapered or thickened toward the end) space portion centering around the central axis (5) and 2 to 4 cylindrical or circular truncated cone shaped space portions provided around the single space portion. Specifically, it is preferable that the centered space portion and its surrounding space portions have different volumes. To be more precise, it is preferable that the volume of the centered space portion is 1.1 to 2 times the volume of a single surrounding space portion. With such an arrangement, various flow rates of the drug solution can be obtained, so that the drug can be administered to deep portions and shallow portions under the skin. Assuming that the height (length) of the 2 to 4 cylindrical portions is L and the diameter of the cross section is D, separated flow is generated in case L/D is equal to or greater than 5, while if L/D is small the loss of current is small but the pressure applied at the tip of the ampoule becomes high so that the ampoule may break. From such a point of view, L/D is preferably between 0.1 and 5, both inclusive, while it may be between 0.5 and 3, both inclusive, between 0.5 and 2, both inclusive, or between 1 and 2, both inclusive .

Since a large head portion is thus provided at the front edge portion (1), by pressing the head portion and by applying the "principle of leverage", a great power can be applied to the joint (4). Therefore, the bonding face can break easily, and a smooth and flat aperture plane can be quickly and easily obtained by applying a lateral torque. It is to be noted that if the length of the front edge portion along the axis is too short, it becomes difficult to apply a lateral force, while if the length is too long, it becomes less portable. Accordingly, for the length of the front edge portion 3 mm - 5×10 mm can be mentioned, while 5 mm - 1×10 mm is more preferable. Also, it is preferable that the space portion (11) connected to the space portion (14) of the joint is provided to a midway of a barrel portion (1b) within the front edge portion (1). Due to such a space portion (11), an opening can be obtained when the joint breaks.

Another preferred embodiment of the hermetical container end according to the first aspect of the present invention is any one of the above-mentioned hermetical container ends, wherein an average outer diameter of the joint (4) is 1/4 - 1/2, preferably 1/3 - 1/2, of a head portion (3a) of the connection (3).

Since the average outer diameter of the joint (4) is within the above-mentioned range, the joint can easily break when a force is applied to the front edge portion.

Another preferred embodiment of the hermetical container end of the present invention is any one of the above-mentioned hermetical container ends, wherein the head portion of the connection has a planar portion which vertically intersects with the central axis (5). It is to be noted that the head portion may be tapered. For such a tapered portion, a height of 1×10⁻¹ mm - 5×10⁻¹ mm, preferably 2×10⁻¹ mm - 4×10⁻¹ mm, can be mentioned, a base diameter of 1 mm - 5x10 mm, preferably 2 mm - 2x10 mm and more preferably 3 mm - 1x10 mm, can be mentioned. Thus, the adhesiveness between the needleless injector and the skin can be increased. The tapered portion means a portion such as a circular truncated cone shaped portion which is tapered towards the end.

Since the head portion of the connection has the planar portion which vertically intersects with the central axis (5), when the joint (4) breaks, it breaks along this planar portion, resulting in a flatter broken surface while preventing a situation where broken pieces are generated. It is to be noted that this planar portion is preferably made coaxial with the central axis (5).

Another preferred embodiment of the hermetical container end according to the first aspect of the present invention is any one of the above-mentioned hermetical container ends, wherein the length of the joint (4) in the direction of the central axis (5) is 1×10⁻³ mm - 1 mm.

When the length of the joint is too long, the broken surface will not be flat, and a possibility of generating broken pieces becomes high. On the other hand, when the length of the joint is too short, it becomes hard to provide the joint. Therefore, for the length of the connection, 1×10⁻³ mm - 1 mm can be mentioned, while 1×10⁻³ mm - 1×10⁻² mm is preferable, and 5×10⁻³ mm - 1×10⁻² mm is more preferable.

Another preferred embodiment of the hermetical container end of the present invention is any one of the above-mentioned hermetical container ends, wherein the front edge portion (1), the joint (4) and the connection (3) are integrally formed, and comprised by a resin composition represented by the following general formula (I) wherein the number average molecular weight is 1×10³ - 1×10⁶ (5×10³ - 5×10⁵ is preferable, 1×10⁴ - 1×10⁵ is more preferable).

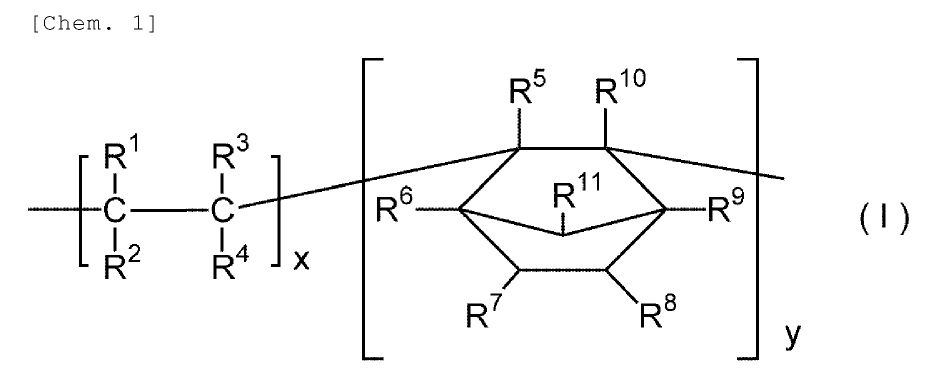

In the above-mentioned formula (I), R¹-R⁴ may be respectively the same or different and represent a hydrogen atom, a C₁-C₃ alkyl group, a hydroxy group, a methoxy group, an ethoxy group, a fluorine atom, a chlorine atom, or a bromine atom; while preferably R¹-R⁴ may be respectively the same or different and represent a hydrogen atom, a methyl group, or a fluorine atom; and more preferably all of R¹-R⁴ are hydrogen atoms. Also, R⁵-R¹¹ may be respectively the same or different and represent a hydrogen atom, a C₁-C₃ alkyl group (a methyl group, an ethyl group, or a propyl group), a hydroxy group, a methoxy group, an ethoxy group, a fluorine atom, a chlorine atom, or a bromine atom, or alternatively R⁷ and R⁸ represent together a C₄-C₆ bicyclo alkyl group which may be substituted with a methyl group, an ethyl group, a methoxy group, an ethoxy group, or a hydroxy group; while preferably R⁵-R¹¹ may be respectively the same or different and represent a hydrogen atom, a methyl group, a hydroxy group, a methoxy group, an ethoxy group, a fluorine atom, or a chlorine atom; and more preferably all of R⁵-R¹¹ are hydrogen atoms. "x" and "y" represent ratio of partial structure composing the ethylene system copolymer where x:y is 1:5 - 5:1, while 1:4 - 4:1 is preferable, 1:3 - 3:1 is more preferable, and 2:3-3:2 is further preferable. Another preferable embodiment of a hermetical container of the present invention is any one of the above-mentioned hermetical container, wherein the glass transition temperature of the resin composition is 140 °C - 180 °C.

The ampoule (20) was actually produced using various materials and experiments for breaking the joint were performed. However, when a material with excessively high crystallinity is used, the joint broke accompanied by generation of broken pieces. On the other hand, when a material with rich ductility or tractility is used, the breaking portion does not break successfully, and a so-calledburr was generated. In the present invention, the hermetical container end is preferably composed only of the above-mentioned resin composition or includes such a resin composition as a main constituent, so that an appropriate hardness is realized and the joint can break smoothly while it is made possible to effectively prevent a situation where the broken pieces are generated. Also, by adopting such a resin composition, sterilization can be facilitated by an immersion in alcohol or an ultraviolet radiation, so that it becomes easy to handle. It is to be noted that for the above-mentioned resin composition the constituent monomers themselves are publicly known, so that by obtaining monomers respectively having double bonds to be mixed according to predetermined proportions and by additive polymerization thereof to be bonded the composition can be easily obtained. Also, the resin composition itself can be purchased from the market.

An example of the hermetical container end of the present invention applied to an ampoule for needleless syringe will now be described. As shown in Fig. 1, an ampoule usable as a needleless syringe is an ampoule provided with an ampoule main body (2) and a hermetical container end (10), wherein the ampoule main body (2) is preferably provided internally with a movable stopper whose diameter is almost the same as the internal surface of the ampoule main body (2). Also, a drug solution is filled preferably in a hermetically-closed state between the movable stopper and a sealed portion (sealed end of the front edge portion).

It is preferable that the ampoule main body (2) has a smooth cylindrical internal surface which enables a gasket (movable stopper) (40) to slide. As an amount of the drug solution to be contained in the ampoule, 1×10⁻¹ ml - 1x10 ml can be mentioned, while 1×10⁻¹ ml - 1 ml is acceptable. It is preferable that the ampoule main body (2) is provided with a shock-absorbing portion for absorbing an impact or an impact sound at a portion adjacent to the hermetical container end. It is to be noted that in order to move the movable stopper, the movable stopper is preferably coupled to a plunger or the like. The movable stopper may be composed of 1 or 2 flexible spheres, for example, for which the inner diameter of the ampoule matches the diameter of the sphere or the diameter of the sphere is 1.01 times - 1.1 times the inner diameter of the ampoule. It is to be noted that the movable stopper is preferably streamlined and the tip becomes narrower towards the ampoule end.

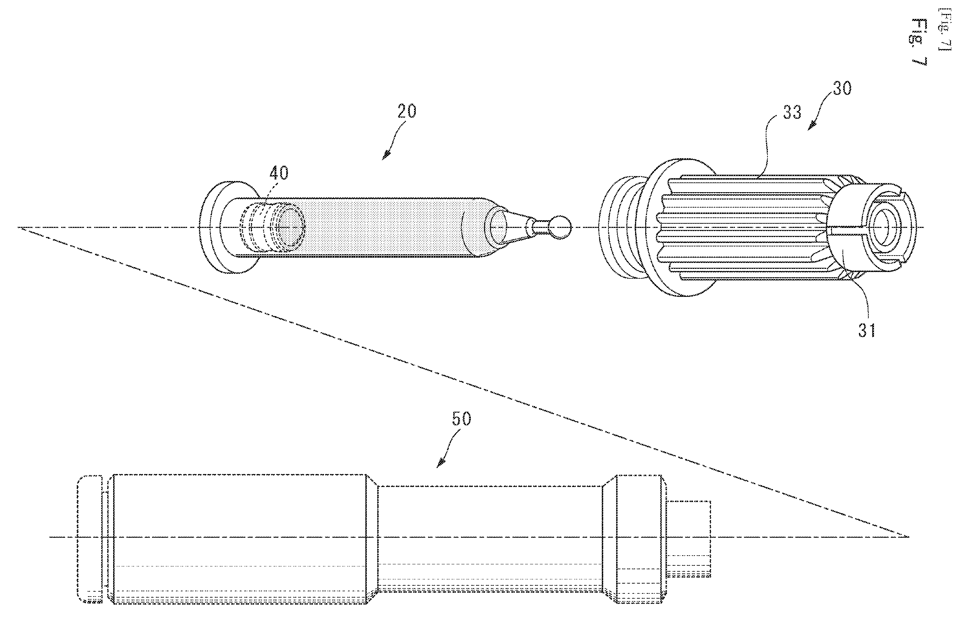

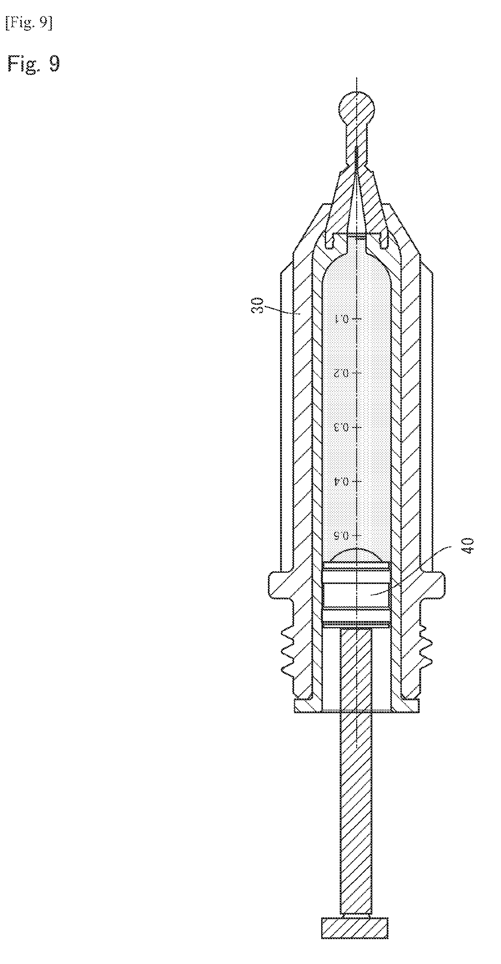

The ampoule of the present invention is applied to a needleless syringe unit, for example. The needleless syringe unit is provided with an ampoule, and ampoule guide, and a releasing mechanism for releasing the drug solution within the ampoule. Fig. 7 is a schematic diagram showing a basic arrangement of the ampoule guide and the ampoule. Fig. 9 shows an overview diagram of an assembled needleless syringe unit. As shown in Fig. 7, the ampoule guide of the present invention basically relates to a needleless syringe system which is provided with an ampoule (20) including a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3) and which is narrower than the front edge portion (1) and the connection (3), a hermetical container end (10) which includes the front edge portion (1), the joint (4) and the connection (3) is formed separately from the main body (2), the front edge portion (1), the joint (4), the connection (3) and the main body (2) are coaxially aligned to have a central axis (5), having sequential space portions (11, 14, 13, 12) extending from the main body (2) to a midway of the front edge portion (1), the joint (4) breaks when a force is applied from a lateral direction with respect to the central axis (5), so that a head portion of the connection (3) which is circular truncated cone shaped is exposed and the space portion (13) of the connection (3) becomes an open end; an ampoule guide (30); a gasket (movable stopper) 40 provided in the ampoule (20) for containing and pushing out the drug solution; and a plunger activating portion (actuator) (50) provided with a push-out mechanism for pushing out the gasket (40). The plunger activating portion (50) is provided with a button (51), which is pressed to release a spring within the syringe main body, so that the movable stopper moves to release the drug solution.

A preferred embodiment of the present invention is related to the above mentioned needleless syringe unit, wherein a hollow cylindrical guard which is formed to be coaxial with the central axis (5) when the ampoule (20) is inserted into the ampoule guide (30) is provided at a tip of the ampoule guide (30), and the front edge portion of the guard remains on inner side of the front edge portion of the connection (3) when the ampoule (20) is inserted into the ampoule guide (30) . When trying to administer a drug solution with a needleless syringe, stability may become an issue in some cases where the direction of the drug administration is unsettled. On the other hand, by providing such a guard as mentioned above, it becomes possible to increase the stability with the guard while pressing the connection (3) or the joint (4) of the ampoule to the skin.

### [Production method]

The hermetical container end, the ampoule and the ampoule guide of the present invention may be produced by a publicly known method for producing an ampoule or the like. Hereinafter, the ampoule of the present invention may be produced as an ampoule or the like provided with a hermetical container end. It is to be noted that molding methods include an injection molding (including a powder injection molding), an extrusion molding, and the like, while an injection molding using a predetermined metal mold can be used preferably. In this case, the above mentioned raw materials may be used as appropriate.

### [Usage]

Usage of the hermetical container end of the present invention will now be described. Basically, by applying a force to the front edge portion (1) from a lateral direction with respect to the central axis (5), the joint (4) breaks, and a head portion of the connection (3) which is circular truncated cone shaped is exposed and the space portion (13) of the connection (3) becomes an open end. This effect can be utilized so that the hermetical container end of the present invention can be applied, for example, to an ampoule for a needleless syringe or the like. Hereinafter, usage in such an embodiment of utilization will be described.

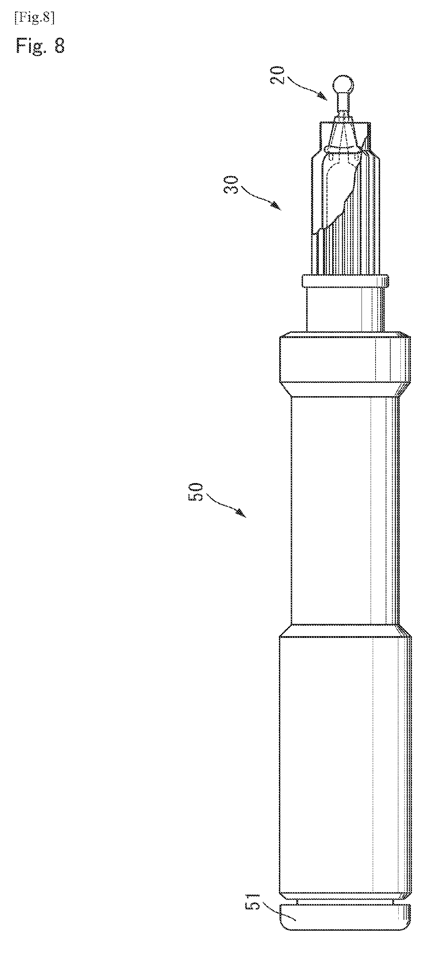

As shown in Fig. 7, the ampoule (20) to be used as a needleless syringe is attached to the ampoule guide (holder: 30). Furthermore, the ampoule guide (30) having attached thereto the ampoule (20) is integrated with the plunger activating portion (50). Fig. 8 is a diagram showing the ampoule as being attached to the ampoule guide and further attached to the plunger activating portion (50). In the state where the ampoule is attached to the ampoule guide, a force is applied to the front edge portion from a lateral direction, or the front edge portion is rotated, so that the joint breaks. Then, the ampoule attached to the ampoule guide is mounted on the needleless syringe main body. Thereafter, the open end of the needleless syringe is applied in close contact with the skin where the drug solution is to be administered.

When the button (51) is subsequently pressed, the gasket (40) included in the ampoule is pushed by the plunger and the like. The drug solution pushed by the movable stopper is emitted through the open end to be administered within the skin.

As for the used needleless syringe unit, the ampoule (20) is removed from the ampoule guide (30) and the ampoule is disposed of.

As a needleless syringe, a publicly known needleless syringe can be used as appropriate. Specific needleless syringes include a needleless syringe described in Japanese Patent Application Laid-Open Publication No. 2003-093508, "a needleless syringe for injecting active principle comprising, from an upstream towards a downstream: a propelling system consisting of an impact wave generating device; a barrier having an upstream surface and a downstream surface, the downstream surface having at least one blind cavity where the active principle can be entered; and an application guide for applying the syringe to a skin of a patient to be treated, **characterized in that** on one hand the barrier is fixed to endure the impact wave while on the other hand the barrier guarantees to favorably propagate the impact wave" as described in Japanese Patent No. 3574433, and "a needleless syringe which injects a drug solution by plunging a piston having readily energized with ahead power in a latched state into the drug solution suctioned from an injection opening of a nozzle comprising: a front cylindrical portion having incorporated therein the nozzle having the injection opening; a rear cylindrical portion which is screwed into the front cylindrical portion and which has a recessed part provided in a longitudinal direction and a recessed part connected to the recessed part in the longitudinal direction on an opposite end to the injection opening to be provided in a circumferential direction of a rotational direction for suctioning of the drug solution; a ball holder integrally provided at the rear cylindrical portion; a plurality of balls in the ball holder; a piston driving rod provided with latching grooves for a part of each of the balls to move in and out and held by the rear cylindrical portion via the ball holder which has the balls included therein; a release switch having a protruding part which is inserted into the recessed part in the longitudinal direction or the recessed part in the circumferential direction of the rear cylindrical portion, and an abutting surface provided so as to abut on the balls in a state where the balls are in the latch grooves, wherein the protruding part can move along the recessed part in the longitudinal direction or the recessed part in the circumferential direction with respect to the rear cylindrical portion; anda compressed spring provided between the rear cylindrical portion and the release switch; **characterized in that** in a state where the balls are not included in the latch grooves, the balls abut on a near side of the abutting surface, so that the release switch is held resisting the compressed spring, and in a state where the balls are included in the latch grooves, the balls surpass the near side of the abutting surface to abut to the abutting surface and hold the piston driving rod, so that as for the release switch, the protruding part of the release switch is guided by the recessed part in the longitudinal direction to move to an end of the recessed part, while the ball holder and the ball rotate, so that the protruding part of the release switch enters the recessed part in the circumferential direction via the abutting surface abutting the balls, whereby a pushing operation of the release switch can be avoided" described in Japanese Patent No. 3560889 can be mentioned.

### [Syringe or administering device]

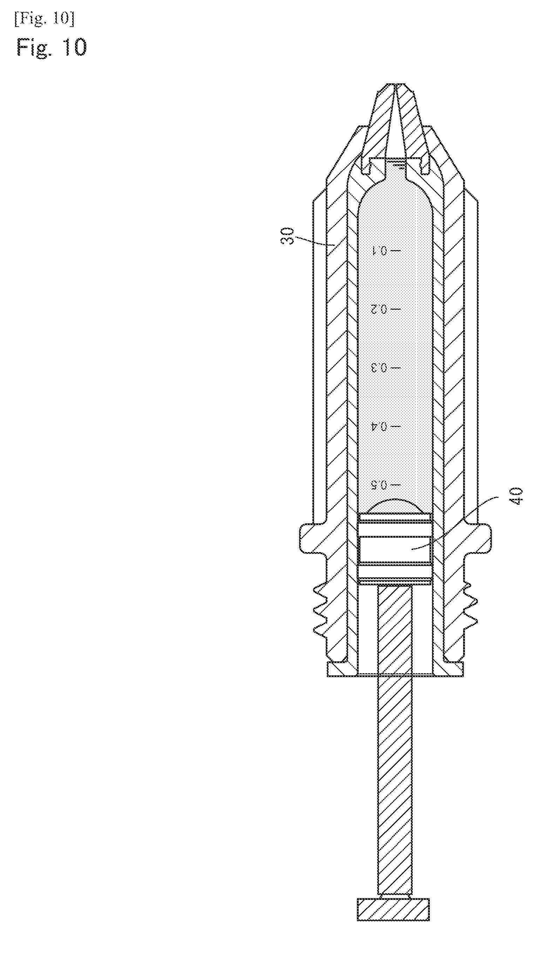

Fig. 9 is a diagram showing a basic arrangement of an administering device. Namely, the present invention can be used in various applications other than a needleless syringe using a plunger activating portion. In a state shown in Fig. 9, the front edge portion is removed. Fig. 10 shows an administering device after the front edge portion has been removed. Then, the drug solution may be accommodated within the ampoule to which a gasket (40) is attached at the bottom, so that by pressing with a finger or the like, the drug within the ampoule can be released outwardly. Tube-type containers are often used for packaging cream or jelly. However, it is difficult to use such tubes in a manner that no content is left behind by squeezing out the entire content. On the other hand, with the ampoule shown in Fig. 10, the entire content can be easily squeezed out by pressing the gasket to the front edge portion.

### [Needle-tipped syringe]

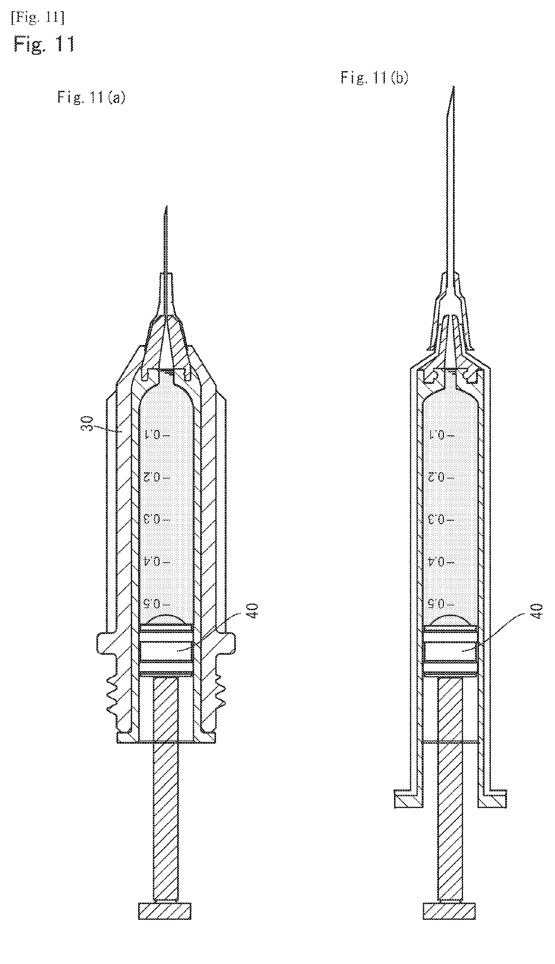

Fig. 11 is a diagram showing an example of a needle-tipped syringe. Fig. 11(A) shows an example of a needle-tipped syringe using an ampoule with an ampoule guide. On the other hand, Fig. 11(B) shows a commercially available syringe which has an ampoule inserted therein. Namely, after the state of Fig. 10 has been achieved, the present invention can be used as a normal syringe by attaching a needle portion. In the example shown in Fig. 10, the ampoule (20), the ampoule guide (30) and the gasket (40) are included. Although it is difficult to attach a needle or an ampoule to a syringe, this example enables a needle or an ampoule to be easily attached since the ampoule guide (30) is provided. Also, since the ampoule guide increases the diameter, an easy-to-hold needle-tipped syringe can be provided. It is to be noted that in case of a needle-tipped syringe, the width of the space portion is preferably made larger than that of the needleless syringe. Also, as shown in Fig. 11(B), the shape of the ampoule may be such that the ampoule can be inserted into a commercially available syringe. Thus, it is made possible to provide needle-tipped syringes including the ampoule of the present invention while using commercially available needle-tipped syringes. Also, the ampoule guide may be capable of accommodating a syringe having an ampoule inserted therein. Thus, the needle and the ampoule are made easy to be attached since the ampoule guide (30) is provided. Also, since the ampoule guide increases the diameter, an easy-to-hold needle-tipped syringe can be provided.

### [Spray diffuser]

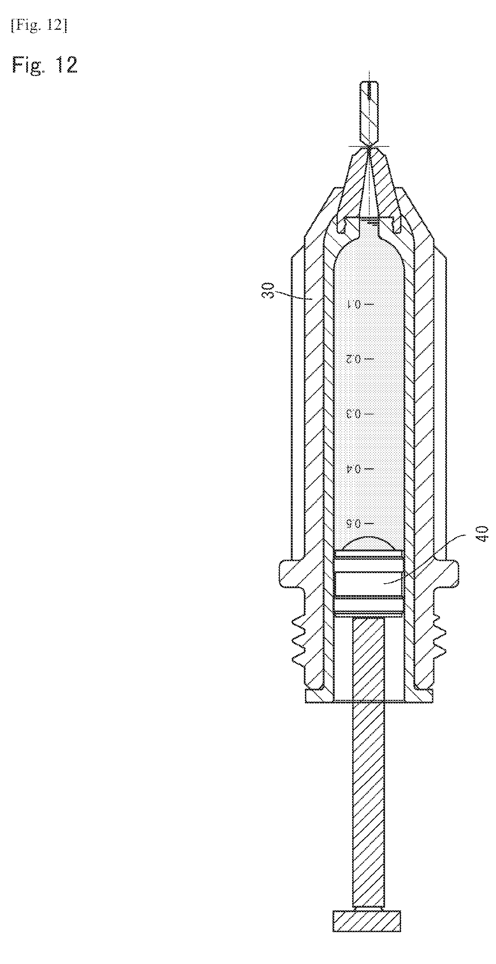

Fig. 12 is a diagram showing an example of a sprayer (diffuser). Specifically, one shown in Fig. 12, which has an inverted front edge portion fitted therein, can be effectively used as a sprayer or the like since the drug solution and the like within the ampoule is diffused in various directions by the front edge portion inserted therein.

### [Industrial Applicability]

As described above, the ampoule of the present invention can be used as a hermetical container end or the like applied to an ampoule for a needleless syringe, so that the ampoule of the present invention can be preferably used in the field of medical equipment. It is obvious that the ampoule of the present invention can be used for other purposes than the needleless syringe in the field of medical equipment as a hermetical container end of a container for a single preparation unit in order to prepare an adequate amount of drug, or preferably used in the fields other than the medical equipment.

## Claims

1. An ampoule (20) comprising:
a front edge portion (1) which is removed when the ampoule (20) is used;
a main body (2) which can accommodate a drug solution;
a connection (3) which connects the front edge portion (1) and the main body (2); and
a joint (4) which joins the front edge portion (1) and the connection (3), and which is narrower than the front edge portion (1) and the connection (3),
a hermetical container end (10) which includes the front edge portion (1), the joint (4) and the connection (3) being formed separately from the main body (2),
the front edge portion (1), the joint (4), the connection (3) and the main body (2) being coaxially aligned to have a central axis (5), having sequential space portions (11, 14, 13, 12) extending from the main body (2) to a midway of the front edge portion (1), the joint (4) breaking when a force is applied from a lateral direction with respect to the central axis (5), so that a head portion of the connection (3) which is circular truncated cone shaped is exposed and the space portion (13) of the connection (3) becomes an open end.

2. The ampoule (20) as claimed in claim 1, wherein
the connection (3) of the hermetical container end (10) comprises:
a hollow cylindrical insertion barrel (6) provided at a portion which is in contact with the main body (2); and
a projection (7) provided on an inner circumferential surface of the insertion barrel (6),
the main body (2) comprises:
a first dent (8) into which the insertion barrel (6) is inserted; and
a second dent (9) into which the projection (7) is inserted,
the insertion barrel (6) is inserted into the first dent (8), and
the projection (7) is inserted into the second dent (9).

3. The ampoule (20) as claimed in claim 1, wherein;
the connection (3) of the hermetical container end (10) comprises:
a first hollow cylindrical insertion barrel (21) provided at a portion which is in contact with the main body (2),
a second hollow cylindrical insertion barrel (22) provided at a portion which is in contact with the main body (2), and having a diameter smaller than that of the first insertion barrel (21), and
a dent provided at a base of the first insertion barrel (21) and the second insertion barrel (22);
the main body (2) comprises:
an insertion barrel (23) of the main body (2) which is inserted to the first insertion barrel (21) and the second insertion barrel (22); and
an insertion barrel front edge portion (24) which is located at a front edge of the insertion barrel (23) of the main body (2) and fits into the dent.

4. The ampoule (20) as claimed in claim 1, wherein;
the hermetical container end (10) has a higher cleavage than that of the main body (2), and
the main body (2) has a higher plasticity than that of the hermetical container end (10).

5. The ampoule (20) as claimed in claim 1, wherein the front edge portion (1) of the hermetical container end (10) has a tip projection (1a) at a tip of the front edge portion (1) which fits into the space portion (14) located at the joint (4).
